(19) Europäisches Patentamt
European Patent Office
Office européen des brevets

(11) Publication number: **0 138 295**
**A1**

# (12) EUROPEAN PATENT APPLICATION

(21) Application number: **84304313.4**

(22) Date of filing: **26.06.84**

(51) Int. Cl.[4]: **C 07 C 67/343**, C 07 C 69/54, C 07 C 69/24, C 07 C 51/353, C 07 C 53/122, C 07 C 57/04, C 07 C 120/00, C 07 C 121/32, C 07 C 121/16

(30) Priority: **11.07.83 GB 8318675**

(43) Date of publication of application: **24.04.85**
**Bulletin 85/17**

(84) Designated Contracting States: **BE DE FR GB IT NL**

(71) Applicant: **IMPERIAL CHEMICAL INDUSTRIES PLC, Imperial Chemical House Millbank, London SW1P 3JF (GB)**

(72) Inventor: **Daniels, James Anthony, 20a Kingsley Road, Frodsham Cheshire (GB)**

(74) Representative: **Stephenson, Kenneth et al, Imperial Chemical Industries PLC Legal Department: Patents PO Box 6, Welwyn Garden City Herts, AL7 1HD (GB)**

(54) **Preparation of monocarboxylic acids, their esters and nitriles.**

(57) A method for the preparation of a compound of the formula:

R-X-Y

wherein R represents hydrogen or a hydrocarbyl radical; X represents a divalent radical of the formula:

$$\underset{\displaystyle CH_2}{\overset{\displaystyle C-}{\|}} \quad \text{or} \quad \underset{\displaystyle CH_3}{\overset{\displaystyle -CH-}{|}}$$

and Y represents -CN or $-COOR^1$ wherein $R^1$ represents hydrogen or an alkyl radical, which comprises reacting a compound of the formula:

$R-CH_2-Y$

wherein R and Y have the above meanings, with methanol at an elevated temperature in the presence of a catalyst comprising alumina and in the substantial absence of formaldehyde or a derivative thereof.

EP 0 138 295 A1

~~KS/13/83~~

TITLE MODIFIED
see front page

CHEMICAL PROCESS

This invention relates to a chemical process and more particularly to a method for the preparation of monocarboxylic acids and the esters and nitriles thereof.

It is known to make α, β-unsaturated acids and the esters and nitriles thereof, often accompanied by the corresponding saturated compounds, by reacting alkanoic acids or the esters or nitriles thereof with formaldehyde in the presence of alkaline catalysts. Thus, US Patent No. 3,247,248 describes the vapour phase reaction between formaldehyde and acetic or propionic acid in the presence of a catalyst such as an alkali metal aluminosilicate to give, respectively, acrylic or methacrylic acid. The formaldehyde may be used in the form of paraformaldehyde or trioxane or a commercially available aqueous or alcoholic solution. A solution of formaldehyde in methanol is specifically

mentioned but it is pointed out that alcoholic solutions of formaldehyde may have some disadvantages such as side reactions when substantial amounts of alcohol are present in the feed mixture. It is also known to use methylal (the dimethylacetal of formaldehyde) in place of formaldehyde itself with the object of avoiding undesirable side reactions.

In addition to the alkanoic acid (or derivatives thereof) and the formaldehyde or methylal, the reaction mixture can contain an alcohol as either a reactant or diluent. Thus, it is shown in UK Patent No. 1,491,183 and in US Patent No. 4,147,718 that methacrylic acid esters may be prepared by reacting methylal with either a propionic acid ester or a mixture of propionic acid and an alcohol.

The use of alcohols, especially lower alkanols, as diluents is described in UK Patent No. 1,428,277 which relates to the production of $\alpha, \beta$-unsaturated acids or esters by reacting an appropriate saturated acid or ester with formaldehyde in the presence of water and a catalyst comprising an alkali metal compound dispersed on a solid carrier. When the starting material is an alkyl carboxylate, the alkanol diluent is said to allow a lower feed molar ratio of alkyl carboxylate to formaldehyde and to inhibit hydrolysis of the feed and product esters. The alkanol diluent remains unchanged at the end of the process and is easily recovered and recycled. The preferred catalyst supports are silica gels and silicas and the Examples describe the preparation of methyl methacrylate and similar esters using potassium hydroxide on silica gel and a methanol/formaldehyde molar ratio of up to 7.5.

Thus, using either formaldehyde or methylal as reactant, it is known to include an alcohol, especially methanol, in the reaction mixture as a solvent, as a diluent or for the in situ conversion of carboxylic acids to esters.

The present invention is based on the surprising discovery that, provided an alumina based catalyst is used, the formaldehyde or methylal can be entirely replaced by methanol. Since formaldehyde and methylal are manufactured by the oxidation of methanol, it is clearly advantageous to proceed direct from the latter.

Accordingly, the invention provides a method for the preparation of a compound of the formula:

$$R-X-Y$$

wherein R represents hydrogen or a hydrocarbyl radical;

X represents a divalent radical of the formula:

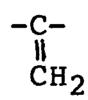 or 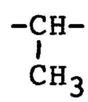

and Y represents -CN or $-COOR^1$ wherein $R^1$ represents hydrogen or an alkyl radical, which comprises reacting a compound of the formula:

$$R-CH_2-Y$$

wherein R and Y have the above meanings, with methanol at an elevated temperature in the presence of a catalyst comprising alumina and in the substantial absence of formaldehyde or a derivative thereof.

The expression "in the substantial absence of formaldehyde or a derivative thereof" means that suitable feed mixtures for use in the reaction contain less than 0.05 mole of formaldehyde, or a derivative thereof such as methylal, per mole of methanol.

In a preferred embodiment of the invention, the feed mixture contains no significant amounts of formaldehyde or derivatives thereof and the method comprises contacting a mixture consisting essentially of methanol and a compound of the formula:

R-X-Y

as hereinbefore defined, with a catalyst comprising alumina at an elevated temperature.

Hydrocarbyl radicals which may be represented by R in the starting material and product include alkyl, cycloalkyl and aryl radicals. Suitable cycloalkyl and aryl radicals include cyclohexyl and phenyl radicals respectively. Suitable alkyl radicals contain up to 8 carbon atoms.

When the starting material and product are esters, alkyl radicals which may be represented by $R^1$ include radicals containing up to 8 carbon atoms.

The invention provides a method of making $\alpha$, $\beta$-unsaturated carboxylic acids, esters and nitriles and/or the corresponding saturated compounds. The ratio of unsaturated to saturated materials in the reaction product depends upon the particular catalyst employed and upon the other reaction conditions. The unsaturated compounds are usually more valuable than the saturated compounds and the conditions are usually selected so as to optimise production of the unsaturated materials. The method of the invention is particularly useful for

preparation of acrylic and methacrylic acids and their ester and nitrile derivatives starting from acetic and propionic acids and their appropriate derivatives. In particular, the invention provides an extremely convenient method of making methyl methacrylate in high selectivity, direct from methanol and methyl propionate.

The catalyst may contain alumina in substantially pure form or in admixture or combination with other materials. Thus, the catalyst may contain alumina, aluminates, amorphous aluminosilicates, zeolites, clays or aluminium phosphates. The preferred catalysts are those in which the alumina, in free or combined form, has been treated with a basic material, for example an alkali metal or alkaline earth metal hydroxide. Accordingly, particularly suitable catalysts include alkali metal aluminates as well as amorphous or crystalline aluminosilicates in their alkali metal or alkaline earth metal forms. Suitable materials may be prepared by known impregnation or ion-exchange techniques followed, if desired, by calcination. The use of the alumina-based catalysts in an alkaline form is particularly useful for avoiding the production of acids in ester preparations. For improved activity and improved selectivity towards $\alpha$, $\beta$-unsaturated products, the catalyst may also contain heavy metals, for example copper, silver, iron or zinc. Such metals may be incorporated by known impregnation or ion-exchange techniques. The catalysts may be calcined at temperatures of up to 700°C although no particular advantage is gained by carrying out the calcination above 500°C. The catalyst may be employed in a fixed, moving or fluidised bed.

The method of the invention is suitably performed at temperatures in the range 200 to 500°C, preferably 300 to 400°C. Atmospheric pressure is preferred although elevated pressures (up to 50 bar) may be employed if desired.

The mole ratio of methanol to acid, ester or nitrile is suitably within the range 0.04:1 to 25:1, preferably 0.1:1 to 10:1 and more especially 0.5:1 to 2:1. Preferably, the reaction is carried out without added solvents and in particular without added water.

The method of the invention may be performed as a batch or continuous process. Preferably, a continuous process is employed, the unreacted starting materials being separated from the product using conventional techniques and recycled to the feed stock.

The invention is illustrated but not limited by the following Examples.

Example 1

5 g of an amorphous ion-exchangeable alumino-silicate material in its $K^+$ form (Decalso T) was treated with an aqueous solution containing 0.6 g of $AgNO_3$. The water was removed from the mixture on a rotary evaporator to produce an even distribution of the salt on the aluminosilicate support material. A portion of the resulting solid was calcined in air at 300°C and found to contain 6.5% Ag by weight.

1 g of the calcined material was packed into a reactor tube and heated to 350°C in a furnace. A mixture of 1.7 parts of methanol to 1 part of methyl propionate was passed over the catalyst diluted in a stream of nitrogen such that the contact time was 18 secs. The product stream was passed through cold traps at -80°C and the liquid condensate was analysed at regular intervals of time by gas chromatography. The conversion

was 20.9% based on methyl propionate. The selectivity to methyl methacrylate was 57.6%, no methyl isobutyrate was detected but propionic acid and propionaldehyde were present in the reaction mixture.

Example 2

A portion of the $AgNO_3$ treated aluminosilicate prepared in Example 1 was calcined in air at 450°C. The procedure described in Example 1 was then followed using a mixture of 1.4 parts of methanol to 1 part of methyl propionate. The conversion was 16.3% based on methyl propionate. The selectivity to methyl methacrylate was 52.0%. A low yield of methyl isobutyrate was found in the first 5 hours of the reaction and propionic acid and propionaldehyde were detected throughout the 28 hour reaction. No loss of catalyst activity was noted throughout this period.

Example 3

A portion of the $AgNO_3$ treated aluminosilicate prepared as in Example 1 was calcined in air at 600°C. The procedure described in Example 1 was then followed using a mixture of 1.4 parts of methanol to 1 part of methyl propionate. The conversion was 19.6% based on methyl propionate. Methyl methacrylate and methyl isobutyrate were found throughout most of the 26 hours of reaction. The methyl isobutyrate predominated in the first 10 hours, methyl methacrylate was the sole $C_5$ product after 22 hours when the selectivity was 61.8%. Propionic acid and propionaldehyde were detected during the reaction.

Example 4

A weight of $AgNO_3$ was deposited onto $K^+$ ion-exchangeable aluminosilicate as described in Example 1 such that the Ag present after calcining at 300° was

10.6% by weight. This material was then tested for catalyst activity as described in Example 1. The conversion was 28.2% based on methyl propionate. The selectivity to methyl methacrylate was 33.3%. No methyl isobutyrate was detected but the main product was propionic acid in 64.0% selectivity.

Example 5

A weight of $AgNO_3$ was deposited onto $K^+$ ion-exchangeable aluminosilicate as described in Example 1 such that the weight of Ag after calcination at 300°C was 2.5%. This material was then tested for catalyst activity as described in Example 1. The conversion was 19.5% based on methyl propionate. The selectivity to methyl methacrylate was 64.6%; no methyl isobutyrate was detected.

Example 6

A weight of $AgNO_3$ was deposited onto $Na^+$ ion-exchangeable aluminosilicate (Decalso S/F) as described In Example 1 such that the weight of Ag after calcination of 450°C was 8.3%. The material was then tested for catalyst activity as described in Example 1. The conversion was found to be 10.2% based on methyl propionate. The selectivity to methyl methacrylate was 85.7%; no methyl isobutyrate was detected.

Example 7

A weight of $Cu(NO_3)_2 3H_2O$ was deposited onto $K^+$ ion-exchangeable aluminosilicate (Decalso T) as described in Example 1 such that the weight of Cu after calcination at 450°C was 3.2%. The material was then tested for catalyst activity as described in Example 1 except that the reaction was carried out at 380°C.

The conversion was found to be 25.0% based on methyl propionate. The selectivity to methyl methacrylate was 65.4%. No methyl isobutyrate was detected but propionic acid and propionaldehyde were found.

A sample of the $K^+$ ion-exchangeable aluminosilicate without added Cu salt was examined for catalyst activity under the same conditions as above. The selectivity to methyl methacrylate was 21.8%. The main product was propionic acid.

<u>Example 8</u>

A weight of $Cu(NO_3)_2 3H_2O$ was deposited onto a $Na^+$ ion-exchangeable aluminosilicate (Decalso S/F) as described in Example 1 such that the weight of Cu after calcination at 300°C was 10.1%. The material was then tested for catalyst activity as described in Example 1 except that the reaction was carried out at 380°C. The conversion was found to be 31.5% based on methyl propionate. The selectivity to methyl methacrylate was 92.1%. No methyl isobutyrate was detected.

A sample of the $Na^+$ ion-exchangeable aluminosilicate without added Cu salt was examined for catalyst activity under the same conditions as above. The conversion was 5.2% based on methyl propionate. The selectivity to methyl methacrylate was 93.3%.

<u>Example 9</u>

A weight of $Cu(NO_3)_2 3H_2O$ was deposited onto a $Na^+$ ion-exchangeable aluminosilicate (Decalso S/F) as described in Example 1, such that the weight of Cu after calcination at 450°C was 9.5%. The material was then tested for catalyst activity as described in Example 1 except that the reaction was carried out at 380°C. The conversion was found to be 27.4% based on methyl propionate. The selectivity to methyl

methacrylate was 79.2%. No methyl isobutyrate was detected. Propionic acid and propionaldehyde were, however, found in the reaction mixture.

Example 10

A weight of $Cu(NO_3)_2 3H_2O$ was deposited onto a $Na^+$ ion-exchangeable aluminosilicate (Decalso S/F) as described in Example 1 such that the weight of Cu after calcination at 600°C was 9.5%. The material was then tested for catalyst activity as described in Example 1 except that the reaction was carried out at 380°C. The conversion was found to be 17.9% based on methyl propionate. The selectivity to methyl methacrylate was 69.6%. A small yield of methyl isobutyrate was detected. Propionic acid, propionaldehyde and isobutyraldehyde were found in the reaction mixture.

Example 11

A weight of $Cu(NO_3)_2 3H_2O$ was deposited onto a $Na^+$ ion-exchangeable aluminosilicate (Decalso S/F) as described in Example 1 such that the weight of Cu was 8%. The material was tested for catalyst activity, without any prior calcination, as described in Example 1 at 380°C. The conversion was found to be 27.0% based on methyl propionate. The selectivity to methyl methacrylate was 85.9%. No methyl isobutyrate was detected.

Example 12

A weight of $Cu(NO_3)_2 3H_2O$ was deposited onto a $Na^+$ ion-exchangeable aluminosilicate (Decalso S/F) as described in Example 1 such that the weight of Cu after calcination of 450°C was 2.4%. The material was tested for catalyst activity as described in Example 1 at 380°C. The conversion was found to be 21.2% based on

methyl propionate. The selectivity to methyl methacrylate was 86.3%. No methyl isobutyrate was detected.

Example 13

A weight of $Fe(NO_3)_2 9H_2O$ was deposited onto a $Na^+$ ion-exchangeable aluminosilicate (Decalso S/F) as described in Example 1 such that the weight of Fe after calcination at 350°C was 8.5%. The material was tested for catalyst activity as described in Example 1 at 380°C. The conversion was found to be 21.4% based on methyl propionate. The selectivity to methyl methacrylate was 87.9%. No methyl isobutyrate was detected. Propionaldehyde, propionic acid and small amounts of $C_6$ and $C_7$ products were observed.

Example 14

A weight of $CuCl_2$ was deposited onto a $Na^+$ ion-exchangeable aluminosilicate (Decalso S/F) as described in Example 1 such that the weight of Cu after calcination at 350°C was 8.1%. The material was tested for catalyst activity as described in Example 1 at 380°C. The conversion was found to be 19.4% based on methyl propionate. The selectivity to methacrylate was 62.1%, selectivity to propionic acid was 33.9%. No methyl isobutyrate was detected.

Example 15

A weight of $Mg(NO_3)_2 6H_2O$ was deposited onto a $Na^+$ ion-exchangeable aluminosilicate (Decalso S/F) as described in Example 1 such that the weight of Mg after calcination at 350°C was 6.1%. The material was tested for catalyst activity as described in Example 1 at 380°C. The conversion was found to be 14.9% based on methyl propionate. The selectivity to methyl methacrylate was 95.7%. No methyl isobutyrate was detected.

Example 16

A weight of $Zn(NO_3)_26H_2O$ was deposited onto a $Na^+$ ion-exchangeable aluminosilicate (Decalso S/F) as described in Example 1 such that the weight of Zn after calcination at 350°C was 8.9%. The material was tested for catalyst activity as described in Example 1 at 380°C. The conversion was found to be 32.2% based on methyl propionate. The selectivity to methyl methacrylate was 53.0%. Methyl isobutyrate was present in low selectivity (7.9%) but the aldehydes propionaldhyde (19.2%) and isobutyraldehyde were detected in fair yield.

Example 17

A weight of $Zn(NO_3)_26H_2O$ and $Cu(NO_3)_23H_2O$ were co-deposited onto a $Na^+$ ion-exchangeable aluminosilicate (Decalso S/F) as described in Example 1 such that the weight of metals after calcination were 4.4% Cu and 4.2% Zn. The material was tested for catalyst activity as described in Example 1 at 380°C. The conversion was found to be 19.6% on methyl propionate. The selectivity to methyl methacrylate was 78.7%. No isobutyrate was present.

Example 18

10 g of neutral alumina (Merck $Al_2O_3$ 90 Aktiv) was treated with an aqueous solution containing 2 g of NaOH. The water was removed from the solution on a rotary evaporator to produce an even distribution of the NaOH on the alumina support. The resulting material was then calcined in air at 450°C for 24 hours and found to contain 10.3% Na. The above material was treated with an aqueous solution containing a weight of $Cu(NO_3)_23H_2O$ as described in Example 1 such that the weight of Cu after calcination at 450°C was 2.0%. The material was tested for catalyst activity as

described in Example 1 at 380°C. The conversion was found to be 47.2% based on methyl propionate. The selectivity to methyl methacrylate was 47.8% and to methyl isobutyrate was 34.2%.

Example 19

A weight of NaOH was deposited onto neutral alumina (Merck $Al_2O_3$ 90 Aktiv) as described in Example 18 such that the weight of Na after calcination at 450°C was 12.1%. The material was tested for catalyst activity as described in Example 1 at 380°C. The conversion was found to be 82.9% based on methyl propionate. The selectivity to methyl methacrylate was 64.2% and to methyl isobutyrate 24.3%. No propionic acid was detected.

Example 20

Using the same catalyst as described in Example 19 the material was tested for catalyst activity as described in Example 1 at 350°C. The conversion was 36.2% based on methyl propionate. The selectivity to methyl methacrylate was 69.5% and to methyl isobutyrate 21.6%. No propionic acid was detected.

A sample of the neutral alumina used in the above experiment was calcined at 450°C without the addition of NaOH. The material was tested for catalyst activity at 350°C. The conversion was 51.2% based on methyl propionate. Considerable quantities of propionic acid were found (72.5% selectivity) and severe methanol decomposition occurred. Methyl methacrylate was the only $C_5$ product detected.

Example 21

A weight of $Cu(NO_3)_2 3H_2O$ was deposited onto neutral alumina (Merck $Al_2O_3$ 90 Akt) as described in Example 1 such that the weight after calcination at

450°C was 9.0%. The material was tested for catalyst activity as described in Example 1 at 350°C. The conversion of methyl propionate was very high producing a wide range of products in the $C_3$-$C_7$ range. Methanol decomposition was very high. Both methyl methacrylate and methyl isobutyrate were detected.

Example 22

A weight of NaOH was deposited onto amorphous silica-alumina (Grace Grade 160 Regular) as described in Example 18 such that the weight of Na after calcination at 450°C was 12.3%. The above material was treated with an aqueous solution containing a weight of $Cu(NO_3)_2 3H_2O$ such that the weight of Cu after calcination at 450°C was 3.8%. The material was tested for catalytic activity at 350°C as described in Example 1. The conversion was found to be 16.3% based on methyl propionate. The selectivity to methyl methacrylate was 93.1%.

Example 23

A weight of $Cu(NO_3)_2 6H_2O$ was deposited onto $Na^+$ 13X zeolite as described in Example 1 such that the weight of Cu after calcination at 450°C was 3.0%. The material was tested for catalytic activity as described in Example 1 at 350°C. The conversion was 39.6% based on methyl propionate. The selectivity to methyl methacrylate was 5.4% and to propionic acid was 82.1%.

Example 24

A weight of $Cu(NO_3)_2 3H_2O$ was deposited onto a $Na^+$ ion-exchangeable alumino-silicate (Decalso S/F) as described in Example 1 such that the weight of the Cu after calcination at 450°C was 10.6%.

1 g of the calcined material was packed into a reactor tube and heated to 350°C in a furnace. A 1:1 mixture of methanol and methyl acetate was passed over the catalyst diluted in a stream of nitrogen such that a residence time of 15 seconds was obtained. The reaction mixture was analysed by gas chromatography at regular intervals. The conversion was 11.8% based on methyl acetate. The selectivity to methyl propionate was 47.5% and to methyl acrylate was 27.0%. Propionic acid, acetic acid, methyl methacrylate and methyl isobutyrate were also detected in low yield.

Example 25

A weight of $Zn(NO_3)_2 6H_2O$ was deposited onto a $Na^+$ ion-exchangeable aluminosilicate (Decalso S/F) as described in Example 1 such that the weight of the Zn after calcination at 450°C was 8.9%.

The material was tested for catalyst activity as described in Example 24 at 380°C. The conversion was 12.6% based on methyl acetate. The selectivity to methyl propionate was 15.5% and to methyl acrylate was 53.0%.

Example 26

A weight of NaOH was deposited onto neutral alumina (Merck $Al_2O_3$ Aktiv 90) as described in Example 18 such that the weight of Na after calcination at 450°C was 12.1%. The material was tested for catalyst activity as described in Example 24 at 380°C. The conversion was 26.7% based on methyl acetate. The selectivity to methyl propionate was 15.0%, to methyl acrylate was 43.0%, to methyl methacrylate was 7.9% and to methyl isobutyrate was 15.9%.

Example 27

A weight of $Zn(NO_3)_2 6H_2O$ was deposited onto a $Na^+$ ion-exchangeable aluminosilicate (Decalso S/F) as described in Example 1 such that the weight of Zn after calcination at 450°C was 8.9%.

1 g of the calcined material was packed into a reactor tube and heated to 350°C in a furnace. A 1:1 mixture of methanol and acetonitrile were passed over the catalyst diluted in a stream of nitrogen such that the residence time of approximately 15 seconds was obtained. The reaction mixture was analysed by gas chromatography at regular intervals. The conversion was 44.2% based on acetonitrile. The selectivity to propionitrile was 61.7% and acrylonitrile 22.7%.

Example 28

A weight of $Cu(NO_3)_2 3H_2O$ was deposited onto a $Na^+$ ion-exchangeable aluminosilicate (Decalso S/F) as described in Example 1 such that the weight of the Cu after calcination at 450°C was 10.6%.

1 g of the calcined material was packed into a reactor tube and heated to 350°C in a furnace. A 3:1 mixture of methanol and propionic acid was passed over the catalyst diluted in a stream of nitrogen such that a residence time of about 15 seconds was obtained. The reaction mixture was analysed at regular intervals by gas chromatography. The reaction produced a complex mixture of products which included methyl propionate, isobutyric acid, methacrylic acid and their esters and aldehydes.

CLAIMS

1. A method for the preparation of a compound of the formula:

$$R-X-Y$$

wherein R represents hydrogen or a hydrocarbyl radical; X represents a divalent radical of the formula:

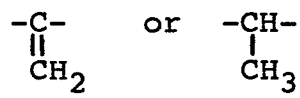

and Y represents -CN or $-COOR^1$ wherein $R^1$ represents hydrogen or an alkyl radical, which comprises reacting a compound of the formula:

$$R-CH_2-Y$$

wherein R and Y have the above meanings, with methanol at an elevated temperature in the presence of a catalyst comprising alumina and in the substantial absence of formaldehyde or a derivative thereof.

2. A method according to Claim 1 wherein R is hydrogen or methyl.

3. A method according to Claim 2 wherein the compound of the formula:

$$R-CH_2-Y$$

is methyl propionate and the reaction product contains methyl methacrylate.

4. A method according to Claim 1 wherein the catalyst comprises alumina that has been treated with a basic material.

5. A method according to Claim 4 wherein the catalyst comprises an alkali metal aluminate or an amorphous or crystalline aluminosilicate in an alkali metal or alkaline earth metal form.

6. A method according to Claim 1 wherein the catalyst contains a heavy metal.

7. A method according to Claim 6 wherein the catalyst contains copper, silver, iron or zinc.

8. A method according to Claim 1 wherein the reaction is performed at a temperature in the range 200 to 500°C.

9. A method according to Claim 8 wherein the reaction is performed at a temperature in the range 300 to 400°C.

European Patent Office

# EUROPEAN SEARCH REPORT

0138295

Application number

EP 84304313.4

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| X | EP - A2 - 0 033 881 (BASF)<br>* Page 3, lines 10-31; examples; claim *<br>-- | 1-9 | C 07 C 67/343<br>C 07 C 69/54<br>C 07 C 69/24<br>C 07 C 51/353<br>C 07 C 53/122<br>C 07 C 57/04<br>C 07 C 120/00<br>C 07 C 121/32<br>C 07 C 121/16 |
| X | GB - A - 1 207 416 (BRITISH PETROLEUM)<br>* Claims *<br>-- | 1,2,4, 6-9 | |
| X | GB - A - 1 207 415 (BRITISH PETROLEUM)<br>* Claims *<br>-- | 1,2,4, 6-9 | |
| A,D | GB - A - 1 491 183 (RÖHM)<br>* Claims; page 2, lines 12-15 *<br>---- | 1-4,6-9 | TECHNICAL FIELDS SEARCHED (Int. Cl.4)<br>C 07 C 57/00<br>C 07 C 67/00<br>C 07 C 69/00 |

The present search report has been drawn up for all claims

| Place of search | Date of completion of the search | Examiner |
|---|---|---|
| VIENNA | 15-10-1984 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document